(19) Europäisches Patentamt

European Patent Office

Office européen des brevets

(11) **EP 4 730 340 A1**

(12) **EUROPEAN PATENT APPLICATION**

(43) Date of publication:
**22.04.2026 Bulletin 2026/17**

(21) Application number: **24207388.0**

(22) Date of filing: **18.10.2024**

(51) International Patent Classification (IPC):
**G16B 30/10** (2019.01)

(52) Cooperative Patent Classification (CPC):
**G16B 30/10; G16B 40/00**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
• **Institut Pasteur de Dakar**
  **BP 220 Dakar (SN)**
• **Berthet, François-Xavier**
  **BP 220 Dakar (SN)**
• **Ashipala, Laimi Sofia Nalitye**
  **10005 Windhoek (NA)**

(72) Inventors:
• **BERTHET, François-Xavier**
  **BP 220 Dakar (SN)**
• **ASHIPALA, Laimi Sofia Nalitye**
  **10005 Windhoek (NA)**
• **SALL, Amadou Alpha**
  **BP 220 Dakar (SN)**

(74) Representative: **Hoffmann Eitle**
**Hoffmann Eitle S.L.U.**
**Paseo de la Castellana 140, 3a planta**
**Edificio LIMA**
**28046 Madrid (ES)**

(54) **METHOD FOR DETERMINING THE PRESENCE OF A PROBE SEQUENCE IN A TARGET NUCLEOTIDE SEQUENCE**

(57) A computer-implemented method for determining the presence of a probe sequence in a target nucleotide sequence by (a) establishing a mapping between each nucleotide of the target sequence and a unique prime number selected from a list of consecutive prime numbers; (b) establishing a mapping between each nucleotide of the probe sequence to be searched and a unique prime number selected from a list of consecutive prime numbers from the list in step a); (c) computing a sequence deconvolution matrix (SDM) using the prime numbers mapped to the nucleotides of the target sequence; (d) individually subjecting each element or component within the SDM matrix of step c) to Euclidean division modulo each of the prime numbers associated with the probe sequence in step b); (e) determining a nucleotide from the probe sequence is present if the remainder is equal to zero and the nucleotide assigned to said position of the SDM matrix has the same nitrogen-containing base as said nucleotide from the probe sequence; and (f) determining that the probe sequence is present in the nucleotide sequence if all the nucleotides have been identified as present in the sequence.

Step ① - Conversion of nucleotides to primes

Target sequence $\rightarrow$ $2 \times 3 \times 5 \times 7 \times 11 \times \ldots \times 53 \times 59 \times 61$
ATGACAGAGCAGCAGTGG

CAGA Probe sequence = $11 \times 13 \times 17 \times 19$

Step ② - Convolution of primes into a matrix

SDM matrix

$$\begin{pmatrix} Gf & 0 & 0 & Af \\ 0 & Tb & Cb & 0 \\ 0 & Ab & Gb & 0 \\ Cf & 0 & 0 & Tf \end{pmatrix} \rightarrow \begin{pmatrix} 37 & 0 & 0 & 62 \\ 0 & 0 & 29 & 0 \\ 0 & 74347 & 330694115 & 0 \\ 451 & 0 & 0 & 159 \end{pmatrix}$$

Step ③ - Euclidean Division Modulo p

| BASE | VALUE | Mod. 11 | Mod. 13 | Mod. 17 | Mod. 19 | Results |
|---|---|---|---|---|---|---|
| Gf | 37 | 4 | 11 | 3 | 18 | Negative |
| Af | 62 | 7 | 10 | 11 | 5 | Negative |
| Tf | 159 | 5 | 3 | 6 | 7 | Negative |
| Cf | 451 | **0** | 9 | 9 | 14 | POSITIVE |
| Gb | 330694115 | 4 | 11 | **0** | 8 | POSITIVE |
| Ab | 74347 | 9 | **0** | 6 | **0** | POSITIVE |
| Tb | 0 | 0 | 0 | 0 | 0 | Negative |
| Cb | 29 | 7 | 3 | 12 | 10 | Negative |

Fig. 1

## Description

## Technical field of the invention

The present invention belongs to the field of bioinformatics, particularly to the field of genomic information processing.

The disclosure relates to ways of representing a sequence of nucleotides as well as uses of these representations. More specifically, the disclosure relates to a method for determining the presence of a probe sequence in a target nucleotide sequence; and to a method for identifying a mutation within one or more nucleotide sequences of interest.

## Background of the invention

In the rapidly evolving fields of molecular diagnostics, genomics, and metagenomics, the ability to accurately and efficiently detect nucleotide sequences is of paramount importance. Such detection is fundamental to a wide range of applications, including sequence-based diagnostics, where identifying specific genetic markers can provide crucial insights into the presence of diseases or predispositions. In genomics and metagenomics, accurate sequence identification allows for the comprehensive analysis of genetic material, enabling the study of complex biological systems, microbial communities, and their interactions. Variant identification, another critical area, relies on precise sequence detection to recognize genetic mutations or polymorphisms, which can have significant implications for understanding disease mechanisms, drug responses, and personalized medicine. Moreover, in the fight against antimicrobial resistance (AMR), detecting specific genetic sequences associated with resistance genes is essential for developing effective treatment strategies and curbing the spread of resistant pathogens.

The current state of the art in DNA detection encompasses a range of sophisticated techniques, with hybridization-based methods being among the most widely used. Techniques such as annealing/melting, PCR, and real-time PCR are foundational in molecular biology, allowing for the amplification and detection of specific DNA sequences with high sensitivity and specificity. PCR (Polymerase Chain Reaction) revolutionized DNA detection by enabling the exponential amplification of target sequences, while real-time PCR, or qPCR, provides quantitative data in real-time, facilitating applications in diagnostics, gene expression analysis, and pathogen detection. Southern and Northern blotting, though more traditional, remain valuable for the detection of DNA and RNA, respectively, using labelled probes that hybridize to target sequences, allowing for the visualization of specific nucleic acids on membranes. Additionally, multiple sequence alignments play a crucial role in comparative genomics and the identification of conserved sequences across different organisms. These alignments allow researchers to detect similarities and differences in DNA sequences, providing insights into evolutionary relationships, functional domains, and potential genetic markers for various applications, including phylogenetics, gene prediction, and variant analysis. Collectively, these techniques represent the current landscape of DNA detection, each contributing uniquely to the field's ability to identify, quantify, and analyse genetic material with precision and accuracy.

However, there is still a need for advancements in technologies that enhance the unmanned and automated identification of genetic sequences hold immense potential to drive innovation and improve outcomes across these diverse and impactful domains.

## Summary of the invention

A first aspect of the invention relates to a method for determining the presence of a probe sequence in a target nucleotide sequence. The method comprises the following steps:

a) establishing a mapping between each nucleotide of the target sequence and a unique prime number selected from a list of consecutive prime numbers comprising as many numbers as nucleotides the target sequence has;

b) establishing a mapping between each nucleotide of the probe sequence to be searched and a unique prime number selected from a list of consecutive prime numbers selected from the list in step a), wherein the prime numbers correspond to the prime numbers mapped to the positions of the target sequence where the probe sequence presence is to be determined;

c) computing a sequence deconvolution matrix (SDM) using the prime numbers mapped to the nucleotides of the target sequence, wherein the SDM matrix comprises elements or components which represent the vertices of a cube; wherein each of the eight vertices of the cube is assigned to a nitrogen-containing base from the subset [A (adenine), C (cytosine), G (guanine), T (thymine)], wherein nitrogen-containing bases T (thymine) and U (uracil) are herein understood as equivalent and thus interchangeable. The SDM matrix is computed by assigning each nucleotide base to a vertex of said cube and computing each of said elements or components of the SDM matrix as the product of all the prime numbers associated to each vertex;

d) individually subjecting each element or component within the SDM matrix of step c) to Euclidean division modulo each of the prime numbers associated with the probe sequence in step b);

e) determining a nucleotide from the probe sequence is present in the target sequence if and only if:

i. the remainder of the Euclidean division of by the prime number mapped to said nucleotide is equal to zero in a position of the SDM matrix, and

ii. the nucleotide assigned to said position of the SDM matrix has the same nitrogen-containing base as said nucleotide from the probe sequence; and

f) determining that the probe sequence is present in the nucleotide sequence if all the nucleotides have been identified as present in the sequence.

In a preferred embodiment of the first aspect of the invention, the method is a computer-implemented method.

In another preferred embodiment of the first aspect of the invention, the list of consecutive prime numbers is a list of the smallest prime numbers.

In another preferred embodiment of the first aspect of the invention, the probe sequence is expressed as the product of the list of prime numbers mapped to the probe sequence.

In another preferred embodiment of the first aspect of the invention, the mapping of step a) is performed in an increasing order of consecutive prime numbers.

In another preferred embodiment of the first aspect of the invention, the probe sequence is a mutation within the target sequence. More preferably, the target sequence is the HIV-1 protease gene. Even more preferably, the probe sequence is a mutation in position 250 of the HIV protease gene.

In another preferred embodiment of the first aspect of the invention, the SDM matrix of step c) is computed according to the following steps:

i. defining a cube using nucleotide bases. Each of the eight vertices of the cube is assigned to a nitrogen-containing base from the subset [A (adenine), C (cytosine), G (guanine), T (thymine)]. It is noted that nitrogen-containing bases T(thymine) and U (uracil) are herein understood as equivalent and thus interchangeable. Each of the vertices of the cube is assigned to a nucleotide base from the subset such that for each vertex, the assigned base is directly connected to every other base type of the subset through an edge,

ii. assigning the first nucleotide base of the target sequence of nucleotides to a vertex of the cube to which the nitrogen-containing base type of the nucleotide has been assigned and sequentially assigning each subsequent nucleotide of the target sequence of nucleotides to a corresponding vertex in the cube, such that the assigned vertex is either directly connected to the vertex of the previous nucleotide base through an edge of the cube or wherein in case the nucleotide base is equal to the previous nucleotide base, the nucleotide base is assigned to the same vertex of the cube as the vertex to which the previous nucleotide base has been assigned to;

iii. identifying all of the nucleotide positions assigned to each vertex of the cube and calculating the product of all of the prime numbers assigned to each of said nucleotide positions, for each vertex of the cube;

iv. assigning each vertex of the cube to an element or component of a matrix with at least as many elements or components as vertices of the cube, and

v. for each of the elements or components of the matrix representing a vertex of the cube, assigning the corresponding product calculated to said vertex computed in step iii).

In a more preferred embodiment of the first aspect of the invention, the matrix is a square matrix. More preferably, the target nucleotide sequence is represented as a 4x4 matrix. Even more preferably, the selected sequence of nucleotides is represented in form of a cube and wherein the vertices of the cube are assigned to the matrix by the 3D projection of the cube on a 2D Euclidean plane defining an inner and external set of nucleotide bases each representing opposing faces of the cube, preferably wherein the projection axis is the one perpendicular to the predetermined initial face.

A second aspect of the invention relates to a method for identifying a mutation within one or more nucleotide sequences of interest. The method comprises the following steps:

a) selecting a reference nucleotide sequence for the one or more nucleotide sequence of interest;

b) computing a hexagonal lattice plot representation (hexaplot) of the reference nucleotide sequence and of the one or more nucleotide sequences of interest. The hexaplot is a mapping of the vertices of a cube, and each of the eight vertices of the cube is assigned to a nitrogen-containing base from the subset [A (adenine), C (cytosine), G (guanine), T (thymine)]. It is noted that nitrogen-containing bases T (thymine) and U (uracil) are herein understood as equivalent and thus interchangeable. The hexaplot is computed by assigning each nucleotide base of its respective sequence to a vertex of said cube and by representing said cube as a union of two planar surfaces of the cube, wherein the two planar surfaces are defined by a cut through a plane comprising 4 vertices of the cube, wherein the 4 vertices are not all connected through edges;

c) characterizing the hexaplot of the reference nucleotide sequence and the hexaplot of each of the one or more nucleotide sequences of interest by using one or more of visual, geometric, and mathematical classifiers;

d) comparing the characterization of each of the hexaplots of the one or more nucleotide sequences of interest with the characterization of the hexaplot of the reference nucleotide sequence; and

e) identifying there is a mutation within the one or more nucleotide sequences of interest if the characterization of the hexaplots of said one or more nucleotide sequences is different to the characterization of the hexaplot of the reference nucleotide sequence.

In a preferred embodiment of the second aspect of the invention, the method is a computer-implemented method.

In another preferred embodiment of the second aspect of the invention, the method is further a method for scoring a mutation within one or more nucleotide sequences of interest, and the method further comprises the step:

f) computing a score based on the difference between the characterization of the hexaplots of said one or more nucleotide sequences and the characterization of the hexaplot of the reference nucleotide sequence.

In another preferred embodiment of the second aspect of the invention, the hexaplot of the reference nucleotide sequence and/or the hexaplot of the one or more of the nucleotide sequences is characterised by:

i. computing the position of each nucleotide on the hexaplot using the Hexagonal Efficient Coordinate System (HECS);
ii. calculating the area of the smallest rectangle enclosing the HexaPlot; and
iii. computing a single numerical value based on the area as calculated on step ii).

More preferably, the single numerical value is computed as the area divided by the length of the nucleotide sequence.

In another preferred embodiment of the second aspect of the invention, the one or more nucleotide sequences of interest are genetic sequences associated with drug resistance.

In another preferred embodiment of the second aspect of the invention, one or more nucleotide sequences of interest are protein genetic sequences.

**Brief description of the drawings**

To enable a better understanding of the present disclosure, and to show how the present disclosure may be carried out, reference will now be made, by way of example only, to the accompanying schematic drawings, wherein:

Figure 1 shows an example of a method for determining the presence of a probe sequence in a target nucleotide sequence according to one or more embodiments of the invention.

Figure 2 shows an example of a method for determining the probe sequence in a target nucleotide sequence, wherein the probe sequence is a mutation according to one or more embodiments of the invention.

Figure 3 shows a diagram of a method for determining the presence of a probe sequence in a target nucleotide sequence according to one or more embodiments of the invention.

Figure 4 shows (A) a representation of a cube in a hexagonal lattice and (B) a geometric method for converting a cube into a hexagonal lattice according to one or more embodiments of the invention.

Figure 5 shows (A) a mapping of the vertices of a genocube to the vertices of a hexagonal lattice and (B) an example of a sequence representation over said mapping according to one or more embodiments of the invention.

Figure 6 shows an example of (A) a random walk, (B) a non-reversing walk, and (C) a self-avoiding walk on a square lattice according to prior art, and (D) an example of a random walk pattern on a hexagonal lattice according to one or more embodiments of the invention.

Figure 7 shows (A) an example of the structure of a protein; (B) the representation of the protein through the mapping of the vertices of a genocube to the vertices of a hexagonal lattice; (C) an example of the structure of an antigen; and (D) the representation of the antigen through the mapping of the vertices of a genocube to the vertices of a hexagonal lattice according to one or more embodiments of the invention.

Figure 8 shows a comparison between the angles of (A) bond angles formed by atoms (carbon, nitrogen, oxygen, and hydrogen) in peptide bonds, and (B) a hexagonal lattice.

Figure 9 shows an example of the the Ramachandran plot of (A) the Psi torsion angle, and (B) the Phi torsion angle of peptide bonds.

Figure 10 shows a comparison between the representation of (A) the wild-type and (B) a mutant version of the katG gene through the mapping of the vertices of a genocube to the vertices of a hexagonal lattice according to one or more embodiments of the invention.

Figure 11 shows (A) a representation of the β-globin gene (HBB) on a hexagonal lattice according to one or more embodiments of the invention and (B) the representation of the 5' end of the HBB gene of the (i) wildtype, (ii) HbS mutant, (iii) HBC mutant and (iv) HbD mutant variants.

Figure 12 shows a diagram of a method for identifying a mutation within one or more nucleotide sequences of interest according to one or more embodiments of the invention.

## Description of the invention

### Definitions

It must be noted that, as used herein, the singular forms "a", "an", and "the", include plural references unless the context clearly indicates otherwise. Further, unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

It is noted that the term "about", as used herein, refers to +/- 30%, preferably +/- 20%, preferably +/- 15%, more preferably +/- 10%, of the indicated referred value.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an aspect or embodiment of the present invention may be substituted with the term "consisting of", though less preferred.

When used herein "consisting of' excludes any element, step, or ingredient not specified in the claim element. When used herein, "consisting essentially of does not exclude materials or steps that do not materially affect the basic and novel characteristics of the claim.

The term "genocube" refers to a cube where each nucleotide is represented twice, on diagonally opposite vertices of the cube.

The term "hexaplot" refers to a hexagonal lattice plot representation of a nucleotide sequence.

The term "symmetric group" refers to the group whose elements are all the bijections from the set to itself, and whose group operation is the composition of functions. In the particular case of this invention, the finite symmetric group $S_4$ defined over a finite set of 4 symbols consists of the permutations that can be performed on the 4 symbols. Since there are n! (n factorial) of such permutation operations, the order (number of elements) of the symmetric group S4 is 4! = 24.

### Description

Each embodiment disclosed herein is contemplated as being applicable to each of the other disclosed embodiments. Thus, all combinations of the various elements described herein are within the scope of the invention. It should also be understood that, unless clearly indicated to the contrary, in any methods claimed herein that include more than one step or act, the order of the steps or acts of the method is not necessarily limited to the order in which the steps or acts of the method are recited

In a previous patent application, EP 4 270 399 A1, we introduced a novel method for encoding any genetic sequence by mapping the sequence's nucleotides as a path along the eight vertices of a cube. Each of the four nucleotides (A, T, C, G) is represented twice, positioned at diametrically opposite vertices on the cube (referred to as the genocube). The nucleotides located on the front face are labeled Gf, Af, Tf, and Cf (where "f" stands for front), while those on the back face are labeled Gb, Ab, Tb, and Cb (with "b" standing for back). This path can be mathematically represented using linear algebra as a 4x4 matrix. By counting the number of times each vertex is crossed, we can generate either a TISA sequence (incrementing the count by 1 for every vertex crossing) or a TDM sequence (where the product of prime numbers reflects the number and sequence of crossings at a given vertex).

Prime numbers possess three critical mathematical properties that are particularly advantageous for the present invention. First, it is a well-established mathematical fact that there is an infinite number of prime numbers, which suggests that we can potentially encode an infinite variety of nucleotide sequences. Second, the well-ordering property of primes ensures that any subset of prime numbers can be ordered sequentially, from the smallest to the largest, which is essential for systematic sequence representation. Third, every integer can be uniquely factored into a product of prime numbers. By leveraging these three properties in combination with the symmetrical properties of SDM (Sequence Deconvolution Matrix) matrices, we have developed a method whereby any target nucleotide sequence can be accurately tested for the presence of a probe sequence.

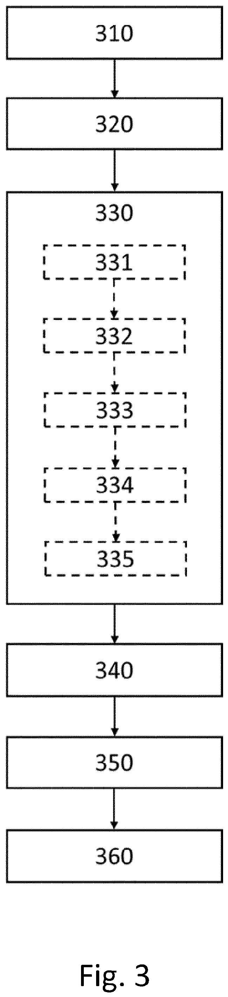

A first aspect of the invention relates to a method for determining the presence of a probe sequence in a target nucleotide sequence. The probe sequence may be any sequence of one or more nucleotides whose presence in the target nucleotide sequence is to be determined. For example, the probe sequence may be mutation, or a sequencing error within the target nucleotide sequence. The method may be regarded as Euclidean Division-based Sequence Identification Method (ED-SIM). As shown in Fig. 3, the ED-SIM method involves the following steps.

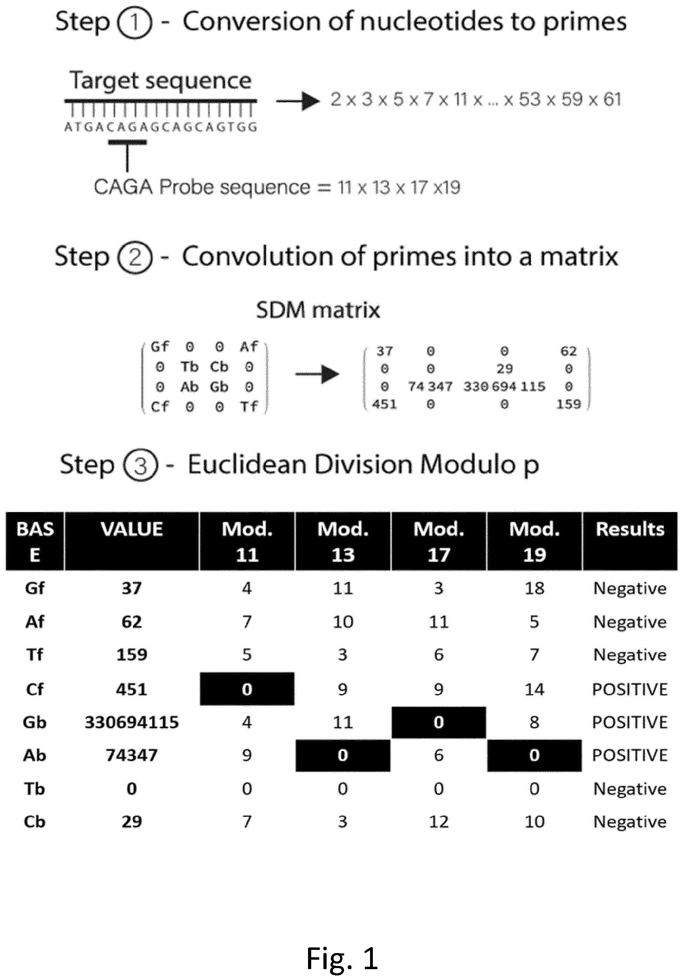

In a first step 310, the method involves establishing a mapping between each nucleotide of the target sequence and a unique prime number selected from a list of consecutive prime numbers comprising as many numbers as nucleotides the target sequence comprises. As shown in Fig. 1, a sequence with 18 nucleotides may be mapped to a set of 18 consecutive prime numbers. For example, and as shown in Fig. 1, step 1, a sequence with 18 nucleotides may be mapped to all the prime numbers comprised between 2 and 61, included.

In a second step 320, the method involves establishing a mapping between each nucleotide of the probe sequence to be searched and a unique prime number selected from a consecutive prime numbers, selected from the list in step 310. The prime numbers correspond to the prime numbers mapped to the positions of the target sequence where the probe sequence presence is to be determined. For example, and as shown in Fig. 1, step 1, since the probe sequence is expected to be found in positions 5 to 8 of the target sequence, the probe sequence is expressed as the product of the $5^{th}$ to $8^{th}$ prime numbers in the target sequence, 11, 13, 17, 19, i.e. $11 \times 13 \times 17 \times 19 = 46189$.

In a third step 330, the method involves computing a sequence deconvolution matrix (SDM) using the prime numbers assigned to the nucleotides of the target sequence. The SDM matrix comprises elements or components which represent the vertices of a cube, wherein each of the eight vertices of the cube is assigned to a nitrogen-containing base from the subset [A (adenine), C (cytosine), G (guanine), T (thymine)]. It is noted that nitrogen-containing bases T (thymine) and U (uracil) are herein understood as equivalent and thus interchangeable. Thus, the SDM matrix is computed by assigning each nucleotide base to a vertex of said cube and computing each of said elements or components of the SDM matrix as the product of all the prime numbers associated to each vertex.

The sequence deconvolution matrix (SDM) is computed based on a matrixial representation of a cube, wherein the cube vertices represents a nitrogen-containing base from the subset [A (adenine), C (cytosine), G (guanine), T (thymine)]. As disclosed in EP 4 270 399 A1, this cubic representation may be referred to as "genocube". Genocube representation is advantageous since any nucleotide sequence can be represented on such genocube. The resulting group (two copies of $S_4$) is isomorphic to the Octahedral symmetry group ($O_h$) of order 48, resulting from the composition ( $S_4 \circ \mathbb{Z}_2$ ) the permutation ($S_4$) and the cyclic ( $\mathbb{Z}_2$ ) subgroups.

For Example, as shown in Fig. 1, step 2, the target sequence of step 1 can be represented as a SDM matrix by computing the product of all the prime numbers associated to each vertex of the genocube.

A possible way of computing the SDM matrix from a genocube will be disclosed in a preferred embodiment of the first aspect of the invention.

In a fourth step 340, the method involves individually subjecting each element or component within the SDM matrix of step 330 to Euclidean division modulo each of the prime numbers associated with the probe sequence in step 320. The skilled person is well versed on Euclidian division, and would easily know how to perform, for each element or component of the SDM matrix, the Euclidean division modulo each of the prime numbers mapped with the probe sequence.

For Example, as shown in Fig. 1, step 3, the remainder of the Euclidean division of the number of each element or component of the SDM matrix of step 2 modulo each of the prime numbers mapped to the probe sequence.

In a fifth step 350, the method involves determining a nucleotide from the probe sequence is present in the target sequence if and only if, the following two conditions are met:

-    the remainder of the Euclidean division of by the prime number corresponding to said nucleotide is equal to zero in a position of the SDM matrix, and
-    the nucleotide assigned to said position of the SDM matrix has the same nitrogen-containing base as said nucleotide from the probe sequence.

For example, as shown in Fig. 1, step 3, the four nucleotides C, A, G, A are determined to be present in the target sequence, as the remainder of the Euclidean division by the prime numbers mapped to the nucleotides of the probe 11, 13, 17 and 19, is zero, and the nucleotides associated to the positions of the SDM matrix have the nitrogen-containing base Cf, Ab, Gb, Ab, respectively.

Lastly, a sixth step 360, involves determining that the probe sequence is present in the nucleotide sequence if all the nucleotides have been identified as present in the sequence. It is noted that when the probe sequence consists of only one nucleotide, step 360 is redundant as by step 350 the presence of the probe sequence can be already determined. Therefore, any method according to the first aspect of the invention wherein the probe sequence consists of only one nucleotide is considered to have complied with step 360 by performing step 350.

Advantageously, the proposed method of the first aspect of the invention enhances the unmanned and automated identification of genetic sequences hold immense potential to drive unmanned, automated genomic surveillance at a significantly reduced cost, which greatly impacts LMIC's (Low- and Middle-Income Countries).

In a preferred embodiment of the first aspect of the invention, the method is implemented through a computer. Thus, it relates to a computer-implemented method.

Advantageously, the present invention being fully realizable and operable on a computer, offers the significant advantage of entirely circumventing traditional hybridization-based methods and sequence-based alignment bioinformatics techniques, which often require extensive human intervention. By enabling unmanned, automated, rapid, and precise identification of genetic sequences, this invention can revolutionize the fields of molecular diagnostics, genomic surveillance, metagenomics, and molecular phylogeny. Its ability to operate independently and efficiently could dramatically streamline and enhance the accuracy of these critical processes, paving the way for more effective and scalable genomic analyses.

In another preferred embodiment of the first aspect of the invention the list of consecutive prime numbers is a list of the smallest prime numbers. Advantageously, this facilitates the computation of the SDM matrix and further operations on the same. Therefore, the method is ensured to be as efficient as possible. When the method is computer-implemented, determining the list of consecutive prime numbers to be a list of the smallest prime numbers further improves the computation speed.

In another preferred embodiment of the first aspect of the invention, the probe sequence is expressed as the product of the list of prime numbers mapped to the probe sequence. Advantageously, this facilitates the encoding of the probe sequence as a single number. Since the product of prime numbers can always be decomposed into its prime numbers, the original sequence can always be recovered. When the method is computer-implemented, expressing the probe sequence as the product of the list of prime numbers mapped to the probe sequence further improves the computation speed, as the prime sequence can be efficiently+ stored and reliably decoded when required.

In another preferred embodiment of the first aspect of the invention, as shown in Fig. 1, the mapping of step a) is performed in an increasing order of consecutive prime numbers.

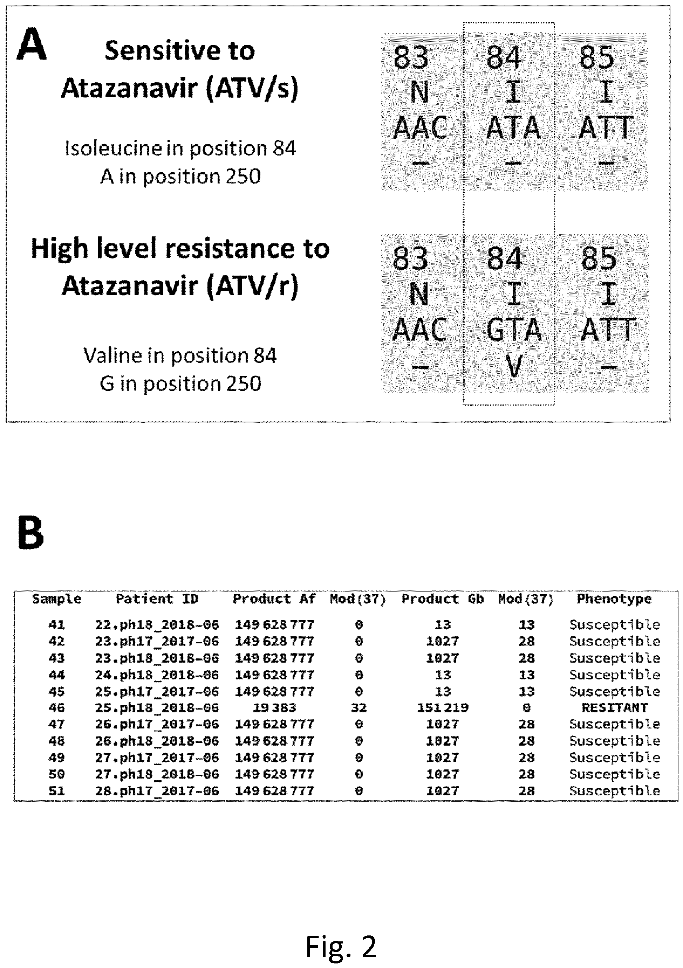

In another preferred embodiment of the first aspect of the invention, the probe sequence is a mutation within the target sequence. As shown in Fig. 2 and further described in Example 2, when the probe sequence is a mutation, such as mutation on position 250 of HIV-1 protease gene, the method according to the first aspect of the invention is able to determine if said mutation is present in said gene. Since the approach is positional, meaning addressing the nature (Gf,Af,Tf,Cf,Gb,Ab,Tb,Cb) of a given mutation, it is particularly advantageous to probe for the presence (Remainder equal to zero) or absence (remainder greater than zero) of highly repetitive sequences, where traditional methods may fail to deliver accurate results.

In a particular preferred embodiment, the target sequence is the HIV-1 protease gene.

In a more particular preferred embodiment, the probe sequence is a mutation in position 250 of the HIV protease gene.

In another preferred embodiment of the first aspect of the invention, the SDM matrix of step 330 is computed according to the following steps as depicted in Fig. 3:

A first step 331 involves defining a cube using nucleotide bases. Each of the eight vertices of the cube is assigned to a nitrogen-containing base from the subset [A (adenine), C (cytosine), G (guanine), T (thymine)]. Nitrogen-containing bases T(thymine) and U (uracil) are herein understood as equivalent and thus interchangeable. Each of the vertices of the cube is assigned to a nucleotide base from the subset such that for each vertex, the assigned base is directly connected to every other base type of the subset through an edge.

Each vertex is assigned a nucleotide base such that for each vertex, the assigned base is directly connected to every other base type through an edge. Therefore, when deciding which nucleotide base is assigned to a vertex, the bases of the vertices directed connected to such vertex through an edge must be considered, and a nucleotide base different from that of the directly connected vertices is assigned. As each vertex is directly connected to other three vertices through an edge, upon completion of the cube definition, any vertex of interest will be directly connected to three vertices through an edge wherein the three vertices have different bases assigned between them and to the base assigned to the vertex of interest.

It is noted that when the nucleotide sequence is represented in the form of a cube, each nucleotide base type is represented twice, on diagonally opposite vertices of the cube. Advantageously, this allows for any nucleotide sequence to be represented on such cube, as in all cases each subsequent nucleotide base can be either assigned to the same vertex as the one the previous nucleotide base has been assigned to, or to a vertex directly connected to the vertex the previous nucleotide base has been assigned to, as each vertex is directly connected to every other base type through an edge.

A second step 332 involves assigning the first nucleotide base of the target sequence of nucleotides to a vertex of the cube to which the nitrogen-containing base type of the nucleotide has been assigned. Then each subsequent nucleotide of the target sequence of nucleotides is sequentially assigned to a corresponding vertex in the cube, such that the assigned vertex is either directly connected to the vertex of the previous nucleotide base through an edge of the cube or wherein in case the nucleotide base is equal to the previous nucleotide base, the nucleotide base is assigned to the same vertex of the cube as the vertex to which the previous nucleotide base has been assigned to.

It is noted that any sequence of DNA, RNA or a set of four modified nucleotides may be therefore represented. It is also noted that in the tetrahedron there are only four vertices, so each vertex has a different nucleotide base assigned. Therefore, the first nucleotide is assigned to the only vertex to which the base type of the nucleotide has been assigned. However, in the cube case, there are eight vertices, so for each nucleotide base, there are two vertices to which the base type of the nucleotide has been assigned. Therefore, the first nucleotide base of the sequence of nucleotides may be assigned to any of the two vertices to which the base type of the nucleotide has been assigned.

In a third step 333, the method involves identifying all of the nucleotide positions assigned to each vertex of the cube and calculating the product of all of the prime numbers assigned to each of said nucleotide positions, for each vertex of the cube.

In a fourth step 334, the method involves assigning each vertex of the cube to an element or component of a matrix with at least as many elements or components as vertices of the cube.

From herein after, the matrix representing the vertices of the cube may be referred to as sequence deconvolution matrix (SDM).

It is noted that several designs may be foreseen when assigning the vertices of the cube or the tetrahedron to a matrix, as multiple choices can be made when deciding how the vertices shall be assigned to each element in the matrix representing a vertex. Moreover, multiple matrix designs can fulfil the requirement of comprising at least as many elements as vertices the cube or the tetrahedron has. Therefore, in the case when the nucleotide sequence is represented in the form of a cube, the sequence may be represented using a 4x4 matrix, wherein half of the elements of the matrix (8) have a vertex of the cube assigned, while the other half (8) doesn't have a vertex of the cube assigned.

Finally, in a fifth step, 335, the method involves, for each of the elements or components of the matrix representing a vertex of the cube, assigning the corresponding product calculated to said vertex computed in step 333.

In a more preferred embodiment, the matrix is a square matrix.

In another more preferred embodiment, the target nucleotide sequence is represented as a 4x4 matrix. In an even further preferred embodiment, the vertices of the of the cube or the tetrahedron are represented on the elements in the diagonals of the matrix. Advantageously, this ensures that whenever the sequence is winded through all the vertices of the cube at least once, the matrix determinant will not be 0. In another even further proffered embodiment, when the nucleotide sequence is represented in the form of a cube, the cube vertices are assigned to a square 4 by 4 matrix wherein the vertices values are assigned to its diagonal elements.

A cube can be represented using isometric 3D projection or projected on the 2D Euclidean plane. Interestingly, the 2D projection transforms the cube into a plannar graph that defines an exterior and an interior set of nucleotide bases as shown in Fig. 1, step 2.

In an ever more preferred embodiment, the selected sequence of nucleotides is represented in form of a cube and wherein the vertices of the cube are assigned to the matrix by the 3D projection of the cube on a 2D Euclidean plane defining an inner and external set of nucleotide bases each representing opposing faces of the cube, preferably wherein the projection axis is the one perpendicular to the predetermined initial face. Advantageously, this allows for a consistent representation of the cube in a square 4x4 matrix wherein the vertices values are assigned to its diagonal elements, as shown for example in Fig. 1. More preferably, the projection axis is the one perpendicular to the predetermined initial face.

In each sequences of nucleotides, a 5' and a 3' end may be defined. In another proffered embodiement of any of the previous embodiments, the received sequence of nucleotides further comprises information indicating the 5' and 3' ends of the sequence and wherein the nucleotides are sequentially assigned to a base in the cube or the tetrahedron in the 5'→3' sense. Advantageously, this further prevents that two different representations of the same sequence may be obtained when no consideration of the starting end is. It is noted that alternatively, the nucleotides may be sequentially assigned to a base in the cube or the tetrahedron in the 3'→5' sense.

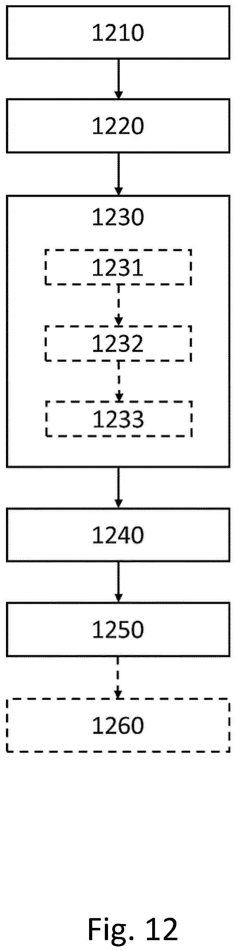

A second aspect of the invention, relates to a method for identifying a mutation within one or more nucleotide sequences of interest. As shown in Fig. 12, the ED-SIM method involves the following steps.

In a first step 1210, the method involves selecting a reference nucleotide sequence for the one or more nucleotide sequence of interest. It is noted that while usually the wildtype variant of a nucleotide sequence may be selected as the reference nucleotide sequence, a certain mutant variant of a nucleotide sequence may be also selected as the reference nucleotide sequence.

In a second step 1220, the method involves computing a hexagonal lattice plot representation of the reference nucleotide sequence and of the one or more nucleotide sequences of interest. From herein after, the hexagonal lattice plot representation will be referred to as "hexaplot".

The hexaplot according to this invention is a mapping of the vertices of a cube; wherein each of the eight vertices of the cube is assigned to a nitrogen-containing base from the subset [A (adenine), C (cytosine), G (guanine), T (thymine)]. It is noted that nitrogen-containing bases T (thymine) and U (uracil) are herein understood as equivalent and thus interchangeable. Thus, each hexaplot is computed by assigning each nucleotide base of its respective sequence to a vertex of said cube and by representing said cube as a union of two planar surfaces of the cube, wherein the two planar surfaces are defined by a cut through a plane comprising 4 vertices of the cube, wherein the 4 vertices are not all connected through.

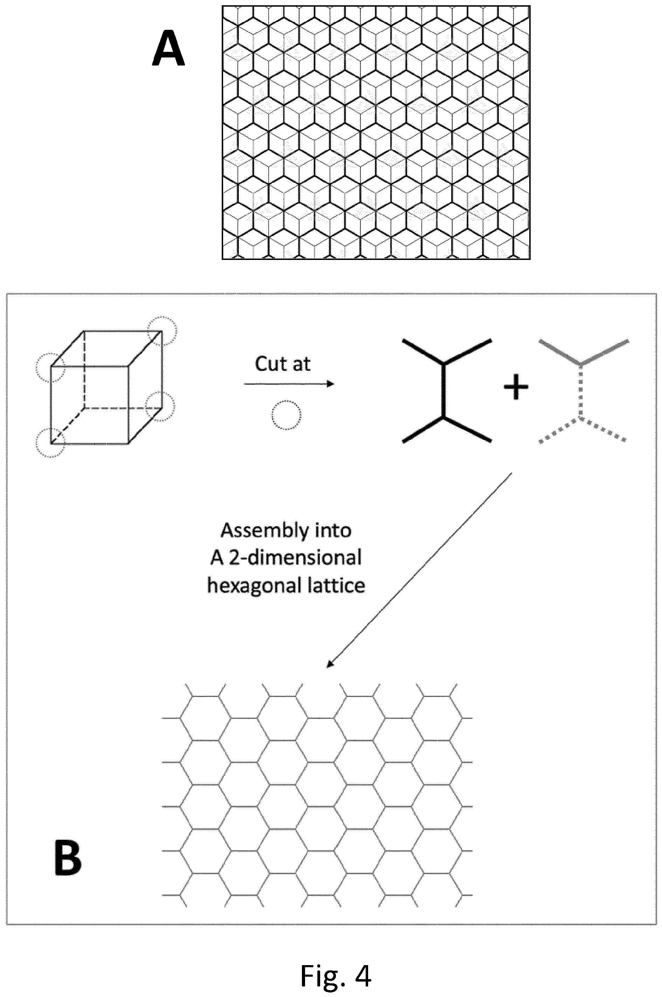

As described in Example 3, and as shown in Fig. 4, when a cube is cut by a plane comprising 4 vertices of the cube, wherein the 4 vertices are not all connected through edges, such as Cf, Tb, Ab, and Tb, it defines two surfaces that can be planarly represented, and which, when combined together, forms a hexagonal lattice. Thus, any cube can be represented as a hexagonal lattice. This optical effect is possible due to a mathematical isomorphism between the projective representation of a three-dimensional cube and the honeycomb lattice.

In a third step 1230, the method involves characterizing the hexaplot of the reference nucleotide sequence and the hexaplot of each of the one or more nucleotide sequences of interest by using one or more of visual, geometric, and mathematical classifiers.

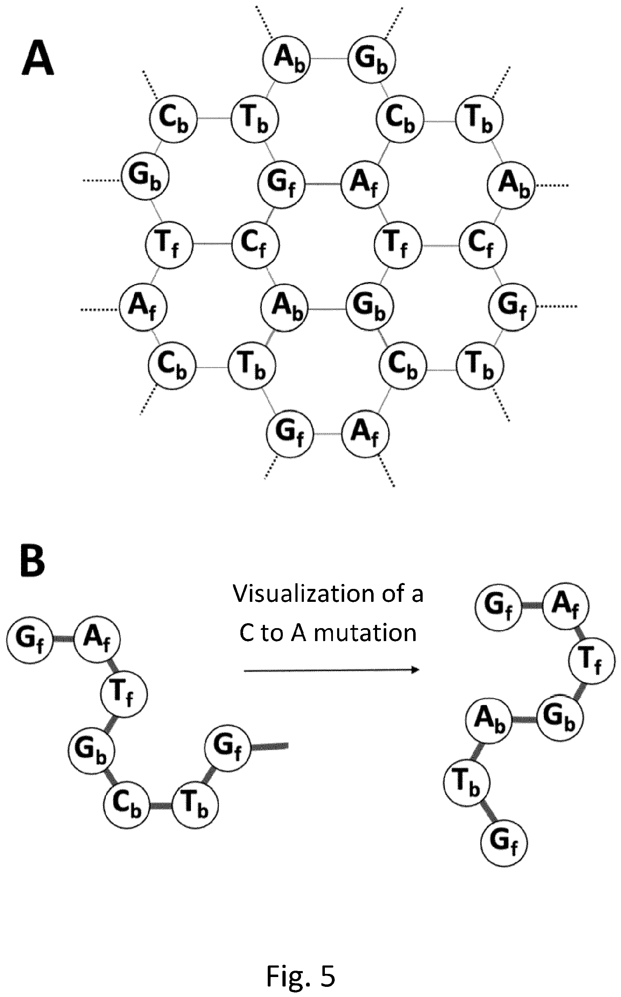

It is noted that, given a hexaplot representation of a sequence, such as that shown e.g. in Figs. 5B, 10, 11A, and 11B, the skilled person may envisage many alternatives in which visual, geometric and/or mathematical classifiers may be used to characterise said sequence. For example, as will be explained later on, the area of a sequence may be used to characterise a sequence.

In step 1240 the method involves comparing the characterization of each of the hexaplots of the one or more nucleotide sequences of interest with the characterization of the hexaplot of the reference nucleotide sequence. Since form the characterization of step 1230 a quantifiable feature (be it visual, geometric or resulting form a mathematical classification) is obtained, this feature can be compared for each of the one or more hexaplots of the one or more nucleotide sequences of interest with that obtained for the reference nucleotide sequence.

Finally, in step 1250 the method involves identifying there is a mutation within the one or more nucleotide sequences of interest if the characterization of the hexaplots of said one or more nucleotide sequences is different to the characterization of the hexaplot of the reference nucleotide sequence.

Advantageously, by mapping the sequence path from the vertices of the Geno-cube to the vertices of the hexagonal lattice (see e.g. Figure 5A), a nucleic acid sequence can be visualized as a path on the honeycomb grid. As depicted in Figure 5B, this method offers a significant advantage: any mutation in the sequence results in a divergence or bifurcation of the path on the hexagonal lattice, making it easier to detect and analyse sequence variations. This innovative approach provides an intuitive way to identify and analyse sequence variations, making it ideal for applications such as polymorphism scoring, phylogenetic analysis, and genome assembly.

In a preferred embodiment of the first aspect of the invention, the method is implemented through a computer. Thus, it relates to a computer-implemented method.

In another preferred embodiment of the second aspect of the invention, the method is further a method for scoring a mutation within one or more nucleotide sequences of interest. As also shown in Fig 12, the method further comprises step 1260, which involves computing a score based on the difference between the characterization of the hexaplots of said one or more nucleotide sequences and the characterization of the hexaplot of the reference nucleotide sequence.

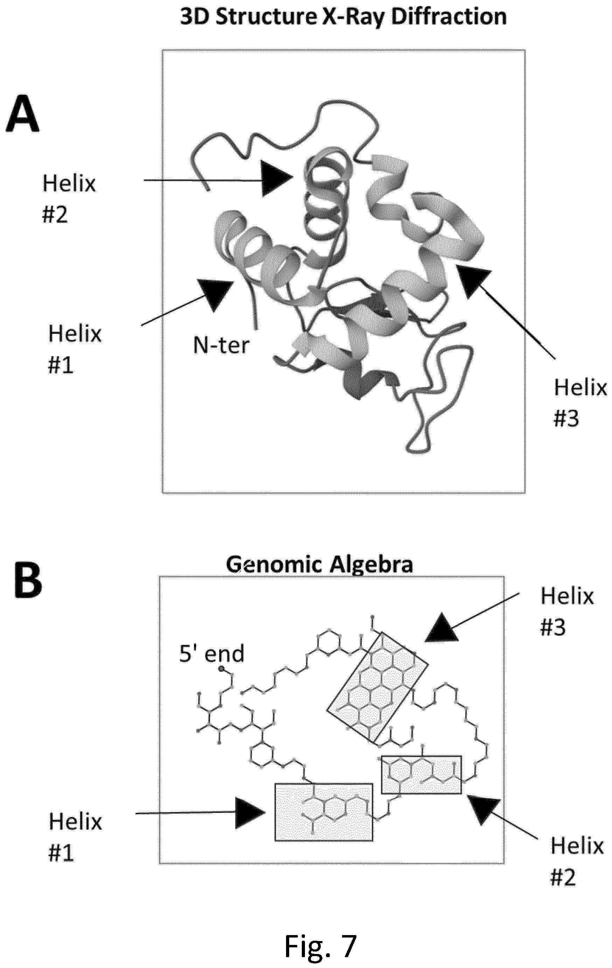

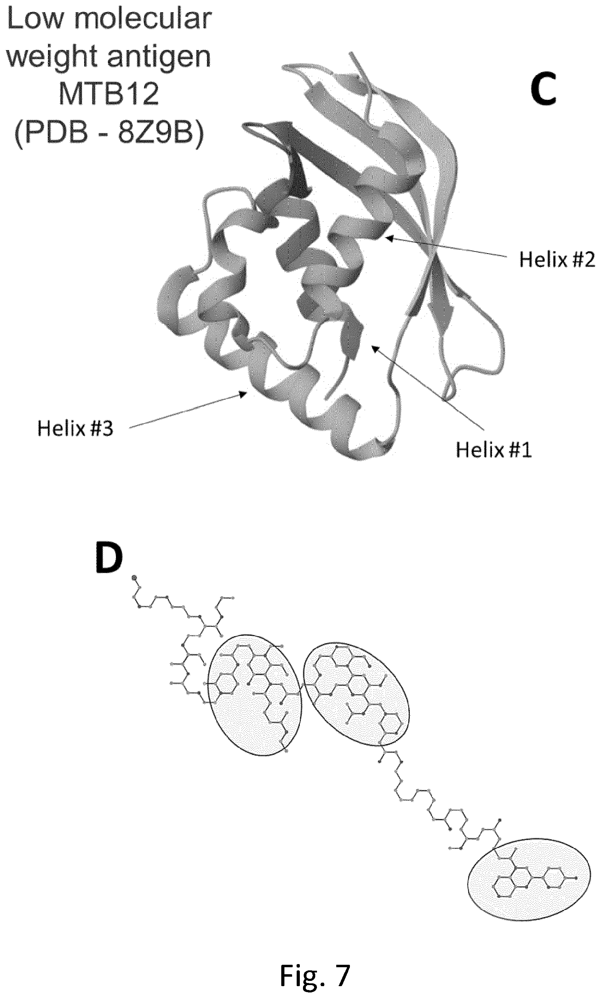

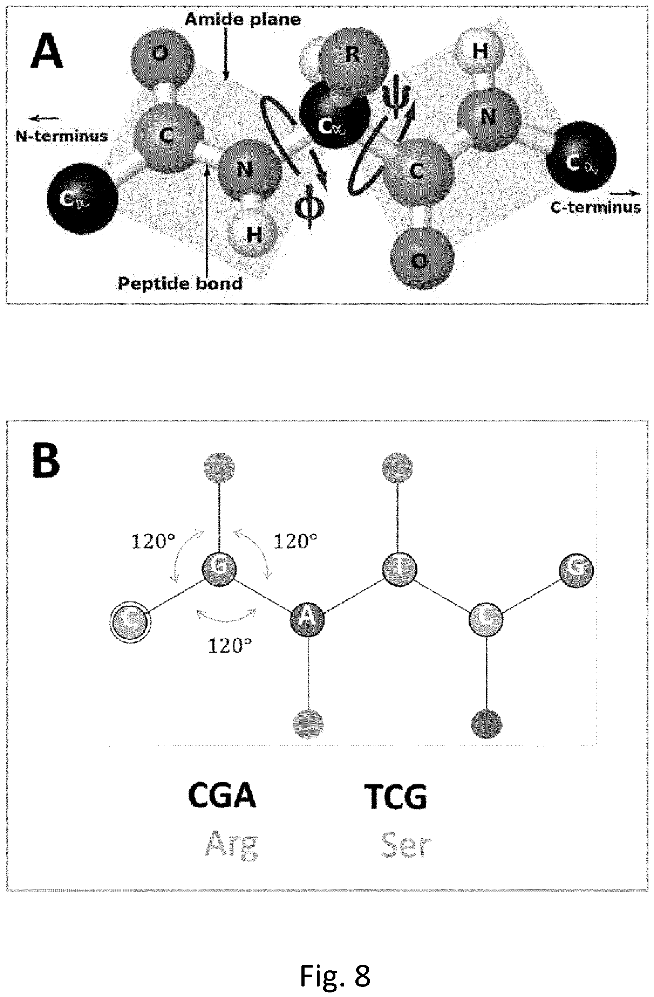

Advantagesouly, this allows for a scoring system that enables classifying and sorting nucleotide sequences accoirdng to their mutation. More particularly, allows for classifying and sorting nucleotide sequences accoirdng to the impact their mutations have on their structural shape. As shown in Example 4, and Figs. 7 to 9, the resemblance between the globular and extended domains observed in the HexaPlot representation of nucleic acid sequences is reminiscent of the structural elements found in protein secondary structures (such as helices and beta-sheets) and tertiary structures (domains). In Examples 5 and 6, practical applications of a scoring system according to the present preferred embodiment of the second aspect of the invention are shown.

Therefore, the present invention allows for scoring the effect of mutations on structural modification of nucleotide sequences.

In another preferred embodiment of the second aspect of the invention, and as shown also in Fig 12, the hexaplot of the reference nucleotide sequence and/or the hexaplot of the one or more of the nucleotide sequences is characterised in step 1230 according to the following steps:

A first step 1231 involves computing the position of each nucleotide on the hexaplot using the Hexagonal Efficient Coordinate System (HECS). The HECS is a well-known system for storing a processing hexagonally sampled images.

In a second step 1232, calculating the area of the smallest rectangle enclosing the HexaPlot.

Finally, in step 1233, computing a single numerical value based on the area as calculated on step 1232.

Advantageously, this method allows for a simple way to characterising an hexaplot, such as that of the reference nucleotide sequence or the one or more nucleotide sequences.

In a more preferred embodiment of the second aspect of the invention, the single numerical value of step 1233 is computed as the area divided by the length of the nucleotide sequence.

In another preferred embodiment of the second aspect of the invention, the one or more nucleotide sequences of interest are genetic sequences associated with drug resistance. As shown in Example 5, the HexaPlot representation can serve as a tool for detecting mutations associated with drug resistance, such as in M. tuberculosis. By providing a clear and visually intuitive distinction between resistant and non-resistant genotypes, it may offer a new approach for rapid, graphical assessment of genetic mutations, complementing existing molecular diagnostics. This capability is particularly promising for enhancing mutation detection in resource-limited settings, where advanced sequencing and computational resources may be less accessible.

In a particular preferred embodiment of the second aspect of the invention, the one or more nucleotide sequences of interest are genes associated with drug resistance to tuberculosis. In a further preferred embodiment, the gene is the katG gene. As shown in Example 5, the proposed method of the second aspect of the invention can accurately assess the resistance to isoniazid (INH) drug for M. tuberculosis treatment.

In another preferred embodiment of the second aspect of the invention, the one or more nucleotide sequences of interest are protein genetic sequences. As shown in Example 6, the HexaPlot representation can serve as a tool for detecting conformational changes in proteins directly from their genetic sequences. This approach enables the creation of novel means of identifying structural variations in proteins by analyzing the visual patterns generated from gene sequences. It opens new avenues for exploring the relationship between genetic mutations and their impact on protein structure, offering potential applications in fields like molecular diagnostics, drug development, and personalized medicine.

In a particular preferred embodiment of the second aspect of the invention, the one or more nucleotide sequences of interest are human haemoglobin genes. As shown in Example 6, the proposed method of the second aspect of the invention can accurately identify weather a mutation in a human haemoglobin gene results in a structural change of the protein.

All of the above are fully within the scope of the present disclosure and are considered to form the basis for alternative embodiments in which one or more combinations of the above-described features are applied, without limitation to the specific combination disclosed above. Particularly, it is noted that all of the method embodiments herein described can be also computer-implemented methods, *mutatis mutandis.*

In light of this, there will be many alternatives which implement the teaching of the present disclosure. It is expected that one skilled in the art will be able to modify and adapt the above disclosure to suit its own circumstances and requirements within the scope of the present disclosure, while retaining some or all technical effects of the same, either disclosed or derivable from the above, in light of his common general knowledge in this art. All such equivalents, modifications or adaptations fall within the scope of the present disclosure.

**Examples**

**Example 1: Detection of genetic sequences**

As illustrated in Figure 1, a genetic sequence may be detected within a target sequence using matrices according to the first aspect of the invention.

The methodology, can be summarized in the following three steps:

Step 1: Establish a mapping between each nucleotide of a target sequence and a corresponding prime number, beginning with the smallest prime, 2. Simultaneously, express the probe sequence to be searched as a product of consecutive primes.

Step 2: The nucleotide sequence is then conceptually folded around the vertices of a genocube, with the corresponding path, represented as a product of prime numbers, and then used to construct a SDM matrix.

Step 3: Each integer value within the SDM matrix is individually subjected to Euclidean division modulo a prime number associated with each of the positions of the probe sequence.

In this approach, a nucleotide from the probe is confirmed to be present in the target sequence if and only if the remainder of the Euclidean division of SDM matrix values by each of the corresponding prime numbers, is equal to zero. As demonstrated in Figure 1, the sequence CAGA, mapped to consecutive primes (11, 13, 17, 19), can be easily identified and reconstituted within the SDM matrix of the target sequence. This method not only enhances the speed of sequence identification but also exemplifies the power and versatility of integrating prime number properties with SDM matrices for molecular diagnostics.

**Example 2 - Identification of HIV mutations conferring resistance to ART**

Antiretroviral therapy (ART) is the cornerstone of HIV treatment. It involves taking a daily combination of HIV medicines, known as an HIV treatment regimen, and is recommended for everyone diagnosed with HIV. Starting ART as early as possible is crucial for managing the infection. While ART does not cure HIV, it enables people with HIV to live longer, healthier lives. By maintaining an undetectable viral load, individuals on ART can reduce the risk of sexually transmitting HIV to their HIV-negative partners to nearly zero. However, over time, people undergoing ART may develop resistance to some of the medications in their regimen. Effective therapy relies on monitoring this resistance through molecular diagnostics and nucleic acid sequencing. These techniques are vital at both the individual and community levels but often require advanced bioinformatics methods that remain challenging to implement in low- and middle-income countries (LMICs).

In this study, we utilized a publicly available sequencing dataset (Abad et al., 2021, [https://pubmed.ncbi.nlm.nih.gov/34941127/]) hosted on the HIV Drug Resistance Database ([https://hivdb.stanford.edu/]). The dataset was employed to evaluate the effectiveness of the Euclidean Division-based Sequence Identification Method (ED-SIM) for detecting mutations that confer resistance to antiretroviral therapy (ART) in individuals living with HIV. This dataset originates from a one-year follow-up of an HIV cohort at an urban center in the Philippines and includes 71 sequences of the HIV-1 protease (pro) gene. Because the sequences reflect real-world data, they include numerous sequencing ambiguities, presenting typical bioinformatics challenges encountered in the field. To address these challenges, all nucleotide ambiguities were systematically interpreted, labeled, and classified based on the guidelines outlined in the following reference: [https://genomevolution.org/wiki/index.php/Ambiguous_nucleotide].

Our study focused on monitoring mutations that confer high-level resistance to the protease inhibitor Atazanavir (ATV). Specifically, we aimed to classify the 71 patient sequences into two categories: ATV/r (resistant) and ATV/s (sensitive). Additionally, we sought to benchmark the performance of the Euclidean Division-based Sequence Identification Method (ED-SIM) against the results published by Abad et al. (2021). As represented in figure 2A, the mutation we were scoring is the amino acid change (I89V) caused by an A to G transition in position 250 of the HIV pro gene.

The dataset was processed using Wolfram Mathematica, following these steps:

(1) importing and converting the sequencing data into FASTA format,

(2) replacing ambiguous nucleotides using the aforementioned reference table,

(3) selecting a target subregion containing the mutational site of interest (22 nucleotides spanning positions 239 to 260),

(4) calculating the path of each sequence within the Geno-cube,

(5) establishing a mapping between each nucleotide of the target sequence and a unique prime number selected from a list of consecutive prime numbers comprising as many numbers as nucleotides the target sequence has, starting with the lowest prime number, 2;

(6) establishing a mapping between the position 250 and the prime number 37. The integer 37 corresponds to the 12th positive prime number, reflecting the value associated with position 250 of the protease gene starting from position 239.

(8) computing a sequence deconvolution matrix (SDM) using the prime numbers mapped to the nucleotides of the target sequence, wherein the SDM matrix comprises elements or components which represent the vertices of a cube; wherein each of the eight vertices of the cube is assigned to a nitrogen-containing base from the subset [A (adenine), C (cytosine), G (guanine), T (thymine)], wherein nitrogen-containing bases T (thymine) and U (uracil) are herein understood as equivalent and thus interchangeable, by assigning each nucleotide base to a vertex of said cube and computing each of said elements or components of the SDM matrix as the product of all the prime numbers associated to each vertex;

(8) individually subjecting each element or component within the SDM matrix of step c) to Euclidean division modulo 37;

(9) determining that a sample comprised a mutation if the remainder of the Euclidean division of by the prime number mapped to said nucleotide is equal to zero in a position of the SDM matrix, and the nucleotide assigned to said position of the SDM matrix has the same nitrogen-containing base as said nucleotide from the probe sequence.

## Results

The analysis identified a single ATV/r mutation among the 71 samples tested.

As shown in Figure 2B, the 46th sample in the dataset, referenced as 28.ph18_2018-06, displayed a remainder of zero when the value for Gb (151219) was divided by 37. Notably, none of the other samples shared this characteristic. This finding aligns perfectly with the results obtained through sequence alignments, as reported by Abad et al. (2021), confirming the accuracy of our method. Interestingly, ED-SIM allows unmanned, machine driven, genomic surveillance and scoring for HIV ART drug resistance and for any genetic mutation relevant for Global Health.

## Example 3 - Representing nucleic acid sequences on a hexagonal lattice (HexaPlot)

Winding genetic sequences around the vertices of the Geno-cube provides a compact and efficient method for managing genetic data, as demonstrated by Topologically Invariant Sequence Array (TISA) and TISA Deconvolution Matrix (TDM) representation. While the 3D representation of nucleic acid sequences on the Geno-cube is machine-readable and suitable for analysis using matrix operations and linear algebra, it is not inherently intuitive for human visualization. To address this limitation, we leveraged the mathematical relationship between a cube (regular hexahedron) and a hexagonal lattice. By transforming a cube into a hexagonal lattice, the three-dimensional data can be reimagined as a two-dimensional path that preserves the spatial relationships of the original structure. This approach simplifies data visualization, making it particularly valuable for genomics, where sequence paths on the Geno-cube can be translated into more visual paths on the hexagonal lattice.

As illustrated in Figure 4A, the perspective of a cube in 3D can be approximated by overlaying two hexagonal lattices (represented as grey and black honeycombs). This optical effect is possible due to a mathematical isomorphism between the projective representation of a three-dimensional cube and the honeycomb lattice. Figure 4B shows a geometric method for converting a cube into a hexagonal lattice. When the cube is cut by a plane comprising 4 vertices of the cube, wherein the 4 vertices are not all connected through edges, such as Cf, Tb, Ab, and Tb, and hexagonal lattice can be formed. By mapping the sequence path from the vertices of the Geno-cube to the vertices of the hexagonal lattice (see Figure 5A), a nucleic acid sequence can be visualized as a path on the honeycomb grid. As depicted in Figure 5B, this method offers a significant advantage: any mutation in the sequence results in a divergence or bifurcation of the path on the hexagonal lattice, making it easier to detect and analyze sequence variations. This innovative approach provides an intuitive way to identify and analyze sequence variations, making it ideal for applications such as polymorphism scoring, phylogenetic analysis, and genome assembly.

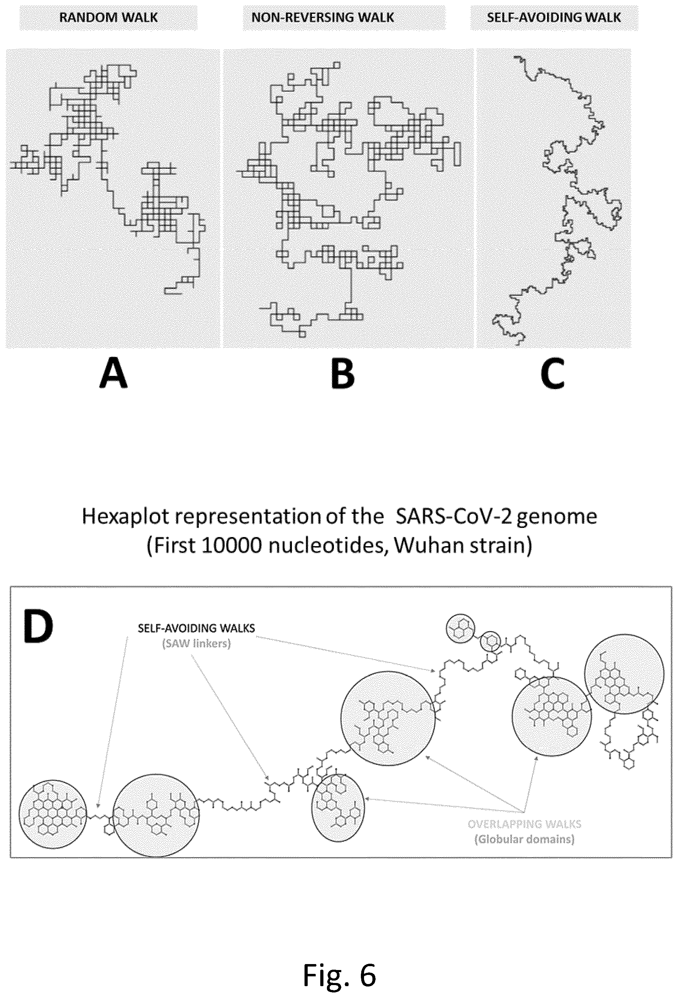

In mathematics, a random walk, also known as a "drunkard's walk," is a stochastic process that describes a path formed by a sequence of random steps within a given mathematical space. Figure 6A illustrates various types of random walks on a square lattice, demonstrating how the characteristics of the walk influence the morphology of the resulting patterns (see Howard C. Berg's Random Walks in Biology). As shown in Figure 6B, when applied to the first 10,000 nucleotides of the SARS-CoV-2 genome, biological sequences generate random walk patterns that display both globular conformational domains (regions of overlapping walks) and extended domains (regions with non-overlapping walks). This behavior has been observed across more than 1,000 independent nucleotide sequences, suggesting that such patterns are a universal and defining characteristic of biological sequences.

### Example 4 - 2D HexaPlots of nucleic acids reflects the 3D structure of proteins

The resemblance between the globular and extended domains observed in the HexaPlot representation of nucleic acid sequences is reminiscent of the structural elements found in protein secondary structures (such as helices and beta-sheets) and tertiary structures (domains). As shown in Figure 7A and 7B, this observation is validated when comparing the positions of structural elements in the Bombyx mori Lysozyme (UniProt P48816) with the locations of globular domains on the HexaPlot of its corresponding gene sequence. Specifically, the three helices illustrated in Figure 7A correspond directly to equivalent regions on the HexaPlot, as depicted in Figure 7B, highlighting a clear alignment between protein structure and the sequence-based HexaPlot representation.

Similarly, as shown in Figure 7C and 7D, this observation is also validated when comparing the positions of structural elements in the antigen MTB12 (PDB - 8Z9B) as described by Ju Hee Han, et al., Novel structure of secreted small molecular weight antigen Mtb12 from Mycobacterium tuberculosis, Biochemical and Biophysical Research Communications, Vol. 717, 2024, 150040, ISSN 0006-291X, https://doi.org/10.1016/i. bbrc.2024.150040; with the locations of globular domains on the HexaPlot of its corresponding gene sequence

This observation is particularly advantageous, as it provides a novel method for predicting the secondary and tertiary structures of proteins directly by analyzing the globular regions (overlapping walks) on the HexaPlot of the corresponding genomic sequences.

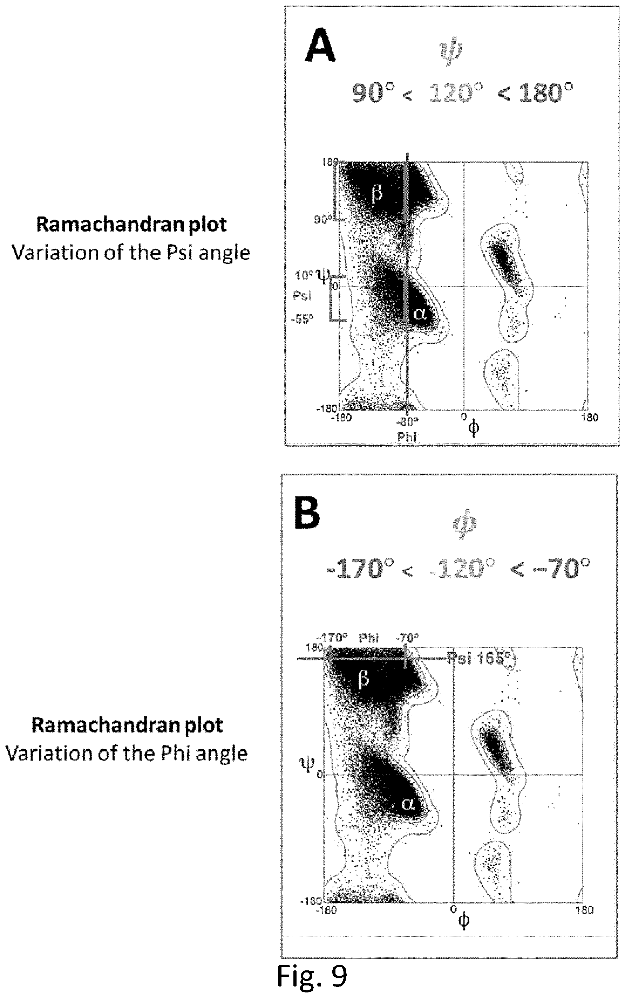

A likely explanation for this phenomenon lies in the geometric similarity between the bond angles formed by atoms (carbon, nitrogen, oxygen, and hydrogen) in peptide bonds, which range from 114° to 123°, and the 120° angles characteristic of a hexagonal lattice (see Figures 7A & 7B). Moreover, the torsion angles Phi ($\phi$) and Psi ($\psi$) of peptide bonds align closely with the 120° angles of the honeycomb lattice, further reinforcing this structural correlation. As illustrated in the Ramachandran plot (Figures 9A and 9B), the 120° angles of the honeycomb lattice fall within the range of rotations defined by the torsion angles Phi ($\phi$) and Psi ($\psi$), supporting this geometric alignment.

### Example 5 - HexaPlot Scoring of antimicrobial resistance in the Tuberculosis bacillus

Tuberculosis (TB) is a highly infectious disease, primarily affecting the lungs, and is caused by the bacterium Mycobacterium tuberculosis. It is transmitted through airborne particles when an infected person coughs, sneezes, or spits, making close contact a significant risk factor for spread. Despite its serious nature, TB is both preventable and curable with proper antibiotic treatment. However, it remains a major global health challenge, with an estimated one-quarter of the world's population infected with latent TB. A critical challenge in the fight against TB is drug resistance, particularly resistance to isoniazid (INH), which is a cornerstone of first-line anti-TB therapy. INH is especially effective during the initial phase of treatment, rapidly reducing the bacterial load. However, resistance to INH poses a significant threat to TB control, as it can progress to multidrug-resistant (MDR) TB, characterized by resistance to both isoniazid and rifampin, another key anti-TB drug. Resistance to INH primarily arises due to mutations in specific genes of the Mycobacterium tuberculosis bacterium, notably katG and inhA. The katG gene encodes an enzyme that activates INH, and mutations in this gene can prevent INH from being converted into its active form. The S315T mutation in the katG gene is the most common mutation associated with INH resistance, significantly reducing the effectiveness of the drug. The emergence of INH resistance is a major concern because it complicates treatment, leading to longer, more complex, and less effective therapeutic regimens. As a result, there is an urgent need for enhanced diagnostic methods to detect resistance early, enabling timely adjustments to treatment plans to prevent the development and spread of MDR-TB. This highlights the importance of developing new approaches to stay ahead of evolving drug resistance and ensure that TB remains a curable disease.

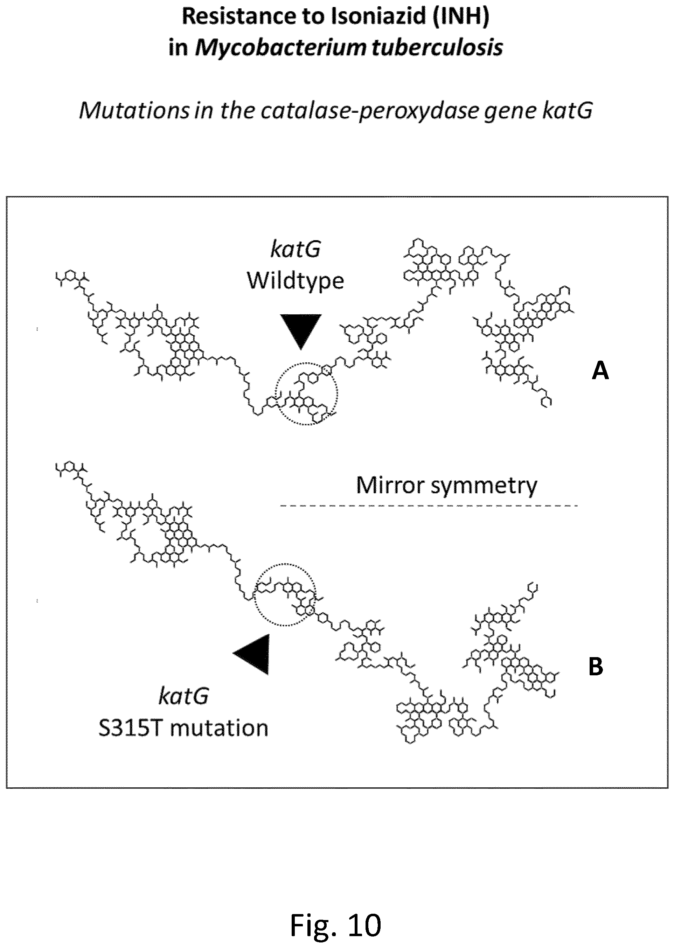

In this study, we explored whether the status of isoniazid (INH) resistance in Mycobacterium tuberculosis could be accurately assessed using the HexaPlot representation. To test this, we obtained both a wild-type and a mutant version of the katG gene (containing the S315T mutation) from the GenBank database. These sequences were then analyzed using Wolfram Mathematica to create their respective HexaPlot representations. As shown in Figure 10A & 10B, the S315T mutation results in a path that is visually distinct from the path of the wild-type sequence. Remarkably, the HexaPlot of the mutant gene displays a pattern that is a near-mirror image of the wild-type pattern, suggesting a fundamental geometric relationship between the mutated and non-mutated sequences. This symmetry indicates that the presence of the S315T mutation can be visually detected and distinguished from the wild-type using this method.

This finding suggests that HexaPlot representation is a valuable tool for detecting mutations associated with drug resistance in M. tuberculosis. By providing a clear and visually intuitive distinction between resistant and non-resistant genotypes, it may offer a new approach for rapid, graphical assessment of genetic mutations, complementing existing molecular diagnostics. This capability is particularly promising for enhancing mutation detection in resource-limited settings, where advanced sequencing and computational resources may be less accessible. Moreover, the use of image analyzing software may allow us to completely automate the determination of drug resistance in TB.

**Example 6** - **HexaPlot analysis of mutations in the human hemoglobin gene**

Detection of human hemoglobin mutations is crucial for diagnosing genetic disorders like sickle cell anemia and thalassemia. These mutations alter the structure of hemoglobin, impacting its ability to transport oxygen efficiently. Techniques such as DNA sequencing, PCR, and high-performance liquid chromatography (HPLC) are commonly used for precise identification of these mutations. Early detection allows for better management and treatment of symptoms, improving patient outcomes. Additionally, genetic screening is valuable for identifying carriers and informing reproductive decisions in at-risk populations. The HbS, HbC, and HbD mutations are genetic variations in the hemoglobin gene that affect the structure and function of hemoglobin, the protein in red blood cells responsible for carrying oxygen. These mutations occur in the β-globin gene (HBB) and result in different forms of abnormal hemoglobin, each associated with varying clinical implications. HbS results from a single point mutation in the HBB gene, where the sixth amino acid in the β-globin chain is changed from glutamic acid to valine (Glu6Val). This mutation is caused by a substitution of adenine to thymine in the DNA sequence (GAG to GTG). HbC results from a different single point mutation in the HBB gene, where the sixth amino acid in the β-globin chain is changed from glutamic acid to lysine (Glu6Lys). This mutation is due to a substitution of adenine for cytosine (GAG to AAG). HbD results from a different mutation in the HBB gene, most commonly involving a substitution of glutamic acid with glutamine at position 121 in the β-globin chain (Glu121Gln).

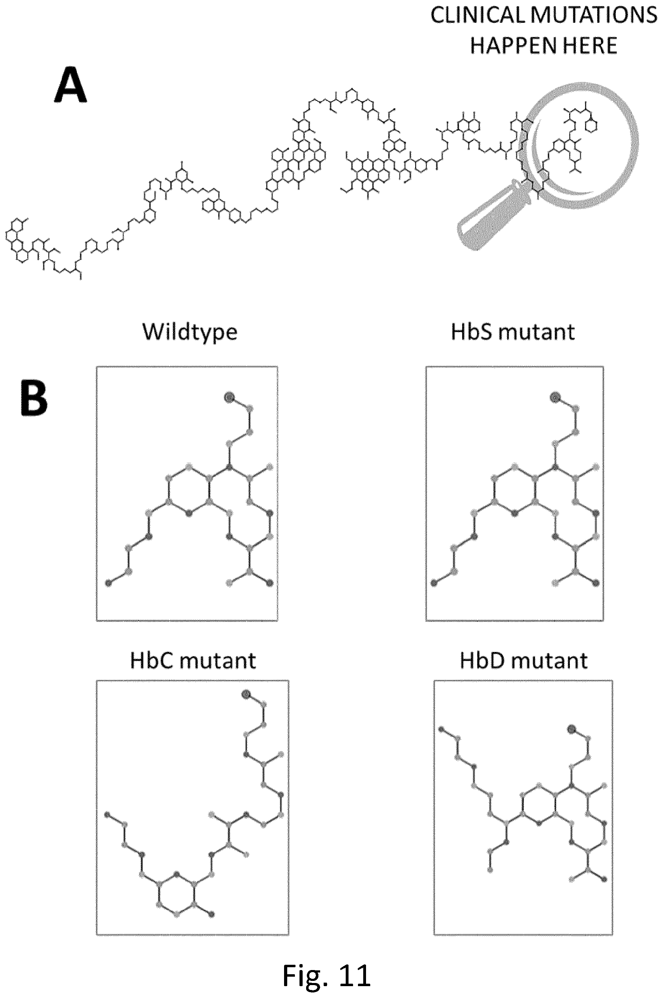

We employed the HexaPlot representation to visualize the paths of both wild-type and mutant versions of the HBB gene, including HbS, HbC, and HbD variants. As illustrated in Figure 11A, all mutations occur near the 5' end of the HBB gene. Notably, the HbS variant exhibits a pattern nearly identical to that of the wild-type sequence, while the HbC and HbD variants display distinct HexaPlot patterns.

This observation is particularly intriguing because the HbS mutation (Figure 11 B) does not alter the three-dimensional structure of the hemoglobin protein itself. Instead, it primarily affects the degree of cross-polymerization between HbS monomers, leading to the characteristic sickling of red blood cells. In contrast, the distinct HexaPlot patterns observed for HbC and HbD suggest changes in the structural conformation of the protein, even at the level of its genetic sequence (Figure 11B).

These findings raise the tantalizing possibility that the HexaPlot representation can serve as a tool for detecting conformational changes in proteins directly from their genetic sequences. This approach could provide a novel means of identifying structural variations in proteins by analyzing the visual patterns generated from gene sequences. It opens new avenues for exploring the relationship between genetic mutations and their impact on protein structure, offering potential applications in fields like molecular diagnostics, drug development, and personalized medicine.

**Claims**

1. A computer-implemented method for determining the presence of a probe sequence in a target nucleotide sequence, comprising the steps of:

 a) establishing a mapping between each nucleotide of the target sequence and a unique prime number selected from a list of consecutive prime numbers comprising as many numbers as nucleotides the target sequence has;
 b) establishing a mapping between each nucleotide of the probe sequence to be searched and a unique prime number selected from a list of consecutive prime numbers selected from the list in step a), wherein the prime numbers correspond to the prime numbers mapped to the positions of the target sequence where the probe sequence presence is to be determined;
 c) computing a sequence deconvolution matrix (SDM) using the prime numbers mapped to the nucleotides of the target sequence, wherein the SDM matrix comprises elements or components which represent the vertices of a cube; wherein each of the eight vertices of the cube is assigned to a nitrogen-containing base from the subset [A (adenine), C (cytosine), G (guanine), T (thymine)], wherein nitrogen-containing bases T (thymine) and U (uracil) are herein understood as equivalent and thus interchangeable, by assigning each nucleotide base to a vertex of said cube and computing each of said elements or components of the SDM matrix as the product of all the prime numbers associated to each vertex;
 d) individually subjecting each element or component within the SDM matrix of step c) to Euclidean division modulo each of the prime numbers associated with the probe sequence in step b);
 e) determining a nucleotide from the probe sequence is present in the target sequence if and only if:

 i. the remainder of the Euclidean division of by the prime number mapped to said nucleotide is equal to zero in a position of the SDM matrix, and
 ii. the nucleotide assigned to said position of the SDM matrix has the same nitrogen-containing base as said nucleotide from the probe sequence; and

 f) determining that the probe sequence is present in the nucleotide sequence if all the nucleotides have been identified as present in the sequence.

2. The computer-implemented method according to claim 1, wherein the list of consecutive prime numbers is a list of the smallest prime numbers.

3. The computer-implemented method according to any one of the previous claims, wherein the probe sequence is expressed as the product of the list of prime numbers mapped to the probe sequence.

4. The computer-implemented method according to any one of the previous claims, wherein the mapping of step a) is performed in an increasing order of consecutive prime numbers.

5. The computer-implemented method according to any one of the previous claims, wherein the probe sequence is a mutation within the target sequence.

6. The computer-implemented method according to claim 5, wherein the target sequence is the HIV-1 protease gene.

7. The computer-implemented method according to claim 6, wherein the probe sequence is a mutation in position 250 of the HIV protease gene.

8. The computer-implemented method according to any one of the previous claims, wherein the SDM matrix of step c) is computed according to the following steps:

 i. defining a cube using nucleotide bases, wherein each of the eight vertices of the cube is assigned to a nitrogen-containing base from the subset [A (adenine), C (cytosine), G (guanine), T (thymine)], wherein nitrogen-containing bases T (thymine) and U (uracil) are herein understood as equivalent and thus interchangeable, and wherein each of the vertices of the cube is assigned to a nucleotide base from the subset such that for each vertex, the assigned base is directly connected to every other base type of the subset through an edge,
 ii. assigning the first nucleotide base of the target sequence of nucleotides to a vertex of the cube to which the nitrogen-containing base type of the nucleotide has been assigned and sequentially assigning each subsequent nucleotide of the target sequence of nucleotides to a corresponding vertex in the cube, such that the assigned vertex is either directly connected to the vertex of the previous nucleotide base through an edge of the cube or wherein in case the nucleotide base is equal to the previous nucleotide base, the nucleotide base is assigned to the same vertex of the cube as the vertex to which the previous nucleotide base has been assigned to;

iii. identifying all of the nucleotide positions assigned to each vertex of the cube and calculating the product of all of the prime numbers assigned to each of said nucleotide positions, for each vertex of the cube;

iv. assigning each vertex of the cube to an element or component of a matrix with at least as many elements or components as vertices of the cube, and

v. for each of the elements or components of the matrix representing a vertex of the cube, assigning the corresponding product calculated to said vertex computed in step iii).

9. The computer-implemented method according to claim 8, wherein the matrix is a square matrix.

10. The computer-implemented method according to claim 9, wherein the target nucleotide sequence is represented as a 4x4 matrix.

11. The method according to claim 10, wherein the selected sequence of nucleotides is represented in form of a cube and wherein the vertices of the cube are assigned to the matrix by the 3D projection of the cube on a 2D Euclidean plane defining an inner and external set of nucleotide bases each representing opposing faces of the cube, preferably wherein the projection axis is the one perpendicular to the predetermined initial face.

## Step ① - Conversion of nucleotides to primes

Target sequence

$\longrightarrow$ 2 x 3 x 5 x 7 x 11 x ... x 53 x 59 x 61

ATGACAGAGCAGCAGTGG

CAGA Probe sequence = 11 x 13 x 17 x19

## Step ② - Convolution of primes into a matrix

### SDM matrix

$$\begin{pmatrix} Gf & 0 & 0 & Af \\ 0 & Tb & Cb & 0 \\ 0 & Ab & Gb & 0 \\ Cf & 0 & 0 & Tf \end{pmatrix} \longrightarrow \begin{pmatrix} 37 & 0 & 0 & 62 \\ 0 & 0 & 29 & 0 \\ 0 & 74347 & 330694115 & 0 \\ 451 & 0 & 0 & 159 \end{pmatrix}$$

## Step ③ - Euclidean Division Modulo p

| BASE | VALUE | Mod. 11 | Mod. 13 | Mod. 17 | Mod. 19 | Results |
|------|-------|---------|---------|---------|---------|---------|
| Gf | 37 | 4 | 11 | 3 | 18 | Negative |
| Af | 62 | 7 | 10 | 11 | 5 | Negative |
| Tf | 159 | 5 | 3 | 6 | 7 | Negative |
| Cf | 451 | **0** | 9 | 9 | 14 | POSITIVE |
| Gb | 330694115 | 4 | 11 | **0** | 8 | POSITIVE |
| Ab | 74347 | 9 | **0** | 6 | **0** | POSITIVE |
| Tb | 0 | 0 | 0 | 0 | 0 | Negative |
| Cb | 29 | 7 | 3 | 12 | 10 | Negative |

# Fig. 1

**A**  Sensitive to
Atazanavir (ATV/s)

Isoleucine in position 84
A in position 250

| 83 | 84 | 85 |
|----|----|----|
| N | I | I |
| AAC | ATA | ATT |
| — | — | — |

**High level resistance to
Atazanavir (ATV/r)**

Valine in position 84
G in position 250

| 83 | 84 | 85 |
|----|----|----|
| N | I | I |
| AAC | GTA | ATT |
| — | V | — |

**B**

| Sample | Patient ID | Product Af | Mod(37) | Product Gb | Mod(37) | Phenotype |
|--------|-----------|-----------|---------|-----------|---------|-----------|
| 41 | 22.ph18_2018-06 | 149 628 777 | 0 | 13 | 13 | Susceptible |
| 42 | 23.ph17_2017-06 | 149 628 777 | 0 | 1027 | 28 | Susceptible |
| 43 | 23.ph18_2018-06 | 149 628 777 | 0 | 1027 | 28 | Susceptible |
| 44 | 24.ph18_2018-06 | 149 628 777 | 0 | 13 | 13 | Susceptible |
| 45 | 25.ph17_2017-06 | 149 628 777 | 0 | 13 | 13 | Susceptible |
| 46 | 25.ph18_2018-06 | 19 383 | 32 | 151 219 | 0 | RESITANT |
| 47 | 26.ph17_2017-06 | 149 628 777 | 0 | 1027 | 28 | Susceptible |
| 48 | 26.ph18_2018-06 | 149 628 777 | 0 | 1027 | 28 | Susceptible |
| 49 | 27.ph17_2017-06 | 149 628 777 | 0 | 1027 | 28 | Susceptible |
| 50 | 27.ph18_2018-06 | 149 628 777 | 0 | 1027 | 28 | Susceptible |
| 51 | 28.ph17_2017-06 | 149 628 777 | 0 | 1027 | 28 | Susceptible |

Fig. 2

Fig. 3

**A**

**B**

Cut at

Assembly into
A 2-dimensional
hexagonal lattice

Fig. 4

Fig. 5

RANDOM WALK

NON-REVERSING WALK

SELF-AVOIDING WALK

A

B

C

Hexaplot representation of the SARS-CoV-2 genome
(First 10000 nucleotides, Wuhan strain)

D

SELF-AVOIDING WALKS
(SAW linkers)

OVERLAPPING WALKS
(Globular domains)

Fig. 6

## 3D Structure X-Ray Diffraction

## Genomic Algebra

Fig. 7

Low molecular
weight antigen
MTB12
(PDB - 8Z9B)

C

Helix #2

Helix #3

Helix #1

D

Fig. 7

**CGA**     **TCG**

Arg      Ser

Fig. 8

**Ramachandran plot**
Variation of the Psi angle

**Ramachandran plot**
Variation of the Phi angle

Fig. 9

**Resistance to Isoniazid (INH)**
**in *Mycobacterium tuberculosis***

*Mutations in the catalase-peroxydase gene katG*

Fig. 10

**A**

CLINICAL MUTATIONS
HAPPEN HERE

**B**

Wildtype

HbS mutant

HbC mutant

HbD mutant

Fig. 11

Fig. 12

Europäisches
Patentamt

European
Patent Office

Office européen
des brevets

## EUROPEAN SEARCH REPORT

**Application Number**

EP 24 20 7388

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 4 270 399 A1 (INST PASTEUR DE DAKAR [SN]) 1 November 2023 (2023-11-01)<br>* paragraphs [0019] - [0021] *<br>* paragraphs [0027] - [0048] *<br>* paragraph [0061] *<br>* paragraphs [0072] - [0082] *<br>* paragraphs [0077] - [0082] *<br>* claims 1-13 *<br>----- | 1-11 | INV.<br>G16B30/10 |
| X | US 2009/307218 A1 (SELLY ROGER [US]) 10 December 2009 (2009-12-10)<br>* claims 1-3,32 *<br>* paragraph [0002] *<br>* paragraph [0036] *<br>* paragraph [0050] - paragraph [0051] *<br>* paragraph [0053] *<br>* paragraph [0152] - paragraph [0153] *<br>----- | 1-11 | |
| X | PROBIR MONDAL ET AL: "Analysis of DNA sequences through local distribution of nucleotides in strategic neighborhoods", ARXIV.ORG, CORNELL UNIVERSITY LIBRARY, 201 OLIN LIBRARY CORNELL UNIVERSITY ITHACA, NY 14853,<br>27 March 2023 (2023-03-27), XP091470256,<br>* sections 2, 3, 5 *<br>----- | 1-11 | **TECHNICAL FIELDS SEARCHED (IPC)**<br><br>G16B |
| X | RANDIC M. ET AL: "On 3-D Graphical Representation of DNA Primary Sequences and Their Numerical Characterization", JOURNAL OF CHEMICAL INFORMATION AND COMPUTER SCIENCES.,<br>vol. 40, no. 5,<br>15 August 2000 (2000-08-15), pages 1235-1244, XP093062951,<br>US<br>ISSN: 0095-2338, DOI: 10.1021/ci000034q<br>* the whole document *<br>-----<br>-/-- | 1-11 | |

The present search report has been drawn up for all claims

| Place of search | Date of completion of the search | Examiner |
|---|---|---|
| Munich | 25 March 2025 | Martínez Cebollada |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

...................................................................................

& : member of the same patent family, corresponding document

EPO FORM 1503 03.82 (P04C01)

**page 1 of 2**

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 5 856 928 A (YAN JOHNSON F [US]) 5 January 1999 (1999-01-05) * the whole document * | 1-11 | |
| A | AKANBASIAM ING. JOSHUA ET AL: "Residue Number System (RNS) Sequence Comparison and Mutation (Error) Analysis", INTERNATIONAL JOURNAL ON BIOINFORMATICS & BIOSCIENCES, vol. 13, no. 02, 27 June 2023 (2023-06-27), pages 1-12, XP093261848, ISSN: 1839-9614, DOI: 10.5121/ijbb.2023.13201 * sections 2, 6, 9 * | 1-11 | |
| A | Anonymous: "Residue number system - Wikipedia", , 9 July 2024 (2024-07-09), XP093261846, Retrieved from the Internet: URL:https://en.wikipedia.org/w/index.php?title=Residue_number_system&oldid=1233477580 * the whole document * | 1-11 | |
| A | AGBEDEMNAB PETER AWON-NATEMI ET AL: "A New Image Encryption and Decryption Technique using Genetic Algorithm and Residual Numbers", 2019 IEEE AFRICON, IEEE, 25 September 2019 (2019-09-25), pages 1-9, XP033788163, DOI: 10.1109/AFRICON46755.2019.9133919 [retrieved on 2020-07-06] * the whole document * | 1-11 | TECHNICAL FIELDS SEARCHED (IPC) |

The present search report has been drawn up for all claims

| Place of search | Date of completion of the search | Examiner |
|---|---|---|
| Munich | 25 March 2025 | Martínez Cebollada |

EPO FORM 1503 03.82 (P04C01)

## ANNEX TO THE EUROPEAN SEARCH REPORT
## ON EUROPEAN PATENT APPLICATION NO.

EP 24 20 7388

This annex lists the patent family members relating to the patent documents cited in the above-mentioned European search report.
The members are as contained in the European Patent Office EDP file on
The European Patent Office is in no way liable for these particulars which are merely given for the purpose of information.

25-03-2025

| Patent document cited in search report | | Publication date | Patent family member(s) | | Publication date |
|---|---|---|---|---|---|
| EP 4270399 | A1 | 01-11-2023 | AU | 2023258659 A1 | 12-12-2024 |
| | | | CN | 119585802 A | 07-03-2025 |
| | | | EP | 4270399 A1 | 01-11-2023 |
| | | | EP | 4515550 A1 | 05-03-2025 |
| | | | WO | 2023209133 A1 | 02-11-2023 |
| US 2009307218 | A1 | 10-12-2009 | EP | 1886226 A2 | 13-02-2008 |
| | | | US | 2009307218 A1 | 10-12-2009 |
| | | | WO | 2006124760 A2 | 23-11-2006 |
| US 5856928 | A | 05-01-1999 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 4270399 A1

**Non-patent literature cited in the description**

- **JU HEE HAN et al.** Novel structure of secreted small molecular weight antigen Mtb12 from Mycobacterium tuberculosis. *Biochemical and Biophysical Research Communications*, 2024, vol. 717, 0006-291X, https://doi.org/10.1016/i.bbrc.2024.150040